# EUROPEAN PATENT SPECIFICATION

(11) **EP 1 060 180 B1**
(45) Date of publication and mention of the grant of the patent: **29.03.2006**
(21) Application number: 99908540.0
(22) Date of filing: 26.02.1999
(51) Int. Cl.: C07D 477/06

(54) **PROCESS FOR SYNTHESIZING CARBAPENEM ANTIBIOTICS**
VERFAHREN ZUR SYNTHESE VON CARBAPENEM-ANTIBIOTIKA
PROCEDE POUR SYNTHETISER DES ANTIBIOTIQUES CARBAPENEMS

(30) Priority: 02.03.1998 US 76829 P; 20.05.1998 GB 9810888
(43) Date of publication of application: 20.12.2000
(73) Proprietor: Merck & Co., Inc., Rahway, New Jersey 07065 (US)
(72) Inventor: WILLIAMS, John, M., Rahway, NJ 07065 (US); BRANDS, Karel, M., J., Rahway, NJ 07065 (US); SKERLJ, Renato, Rahway, NJ 07065 (US); HOUGHTON, Peter, Rahway, NJ 07065 (US)
(74) Representative: Horgan, James Michael Frederic
(86) International application number: PCT/US1999/004321
(87) International publication number: WO 1999/045010

(56) References cited:
- EP-A- 0 533 149
- EP-A1- 0 472 062
- US-A- 5 478 820

## Description

### BACKGROUND OF THE INVENTION

In the past purification and isolation of the compound of formulae I and II were achieved via a combination of several operations: extractions using solvents such as dichloromethane to remove residual organic solvents, chromatography using hydrophobic resins, nanofiltration for concentration of the process stream followed by crystallization of the pure drug. Several of these operations require high capital expenditure. In addition, the time-cycle of such a process is relatively long compromising the quality of the carbapenem product.

The object of the present invention is to teach a much simplified isolation protocol of the compound of formulae I and II via extractions with an alcohol with or without containing an appropriate ion-pairing reagent which concomitantly concentrates the carbapenem compounds followed by a direct crystallization of the product. The present invention is a more practical, efficient, safer and cost effective process because it eliminates the time consuming (and capital intensive) column purification and nanofiltration steps which can compromise the quality of the carbapenem compound due to degradation.

These and other objects will be apparent from the teachings contained herein.

### SUMMARY OF THE INVENTION

1. A process for the direct crystallization of a compound of the formula I: 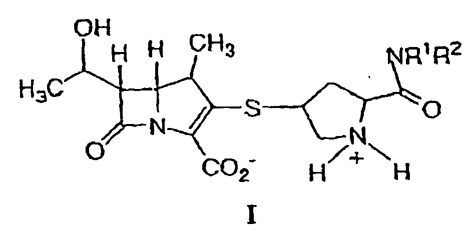 or a pharmaceutically acceptable salt thereof,
   wherein R¹ and R² represent H, C₁₋₁₀ alkyl, aryl or heteroaryl, or substituted C₁₋₁₀ alkyl, aryl or heteroaryl , comprising
   extracting a solution containing a crude compound of formula 1 or Ia: 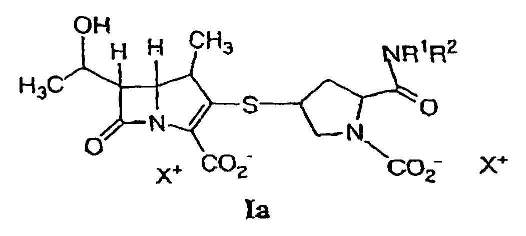 or a pharmaceutically acceptable salt thereof, wherein each X+ is a charge balancing group, and R1 and R2 are as described above with a C4-10 alcohol, collecting and crystallizing the resulting aqueous phase to produce a crystalline compound of formula I ; where "aryl" refers to phenyl or naphthyl, the term "heteroaryl" refers to a monocyclic aromatic hydrocarbon group having 5 or 6 ring atoms, or a bicyclic aromatic group having 8 to 10 atoms, containing at least one heteroatom, O, S or N, in which a carbon or nitrogen atom is the point of attachment, and in which one additional carbon atom is optionally replaced by a heteroatom selected from O or S, and in which from 1 to 3 additional carbon atoms arc optionally replaced by nitrogen heteroatoms, and substituted alkyl, aryl and heteroaryl are substituted with from 1-3 groups selected from the group consisting of: halo, hydroxy, cyano, acyl, acylamino, aralkoxy, alkylsulfonyl, arylsulfonyl, alkylsulfonylamino, arylsulfonylamino, alkylaminocarbonyl, alkyl, alkoxy, aryl, aryloxy, amino, alkylamino, dialkylamino, carboxy and sulfonylamino.

### DETAILED DESCRIPTION OF THE INVENTION

Carbapenems can be prepared by coupling the appropiate sidechain to a protected enolphosphate (3). In this reaction a base and solvent are used. The resulting crude product can then be deprotected to give the desired carbapenem in its crude form. In order to isolate this product in pure form via crystallization sideproducts and solvents need to be removed and the concentration of the product increased. In the past this has been typically achieved via a combination of extraction, column chromatography and nanofiltration.

It has now been discovered that the column chromatography and nanofiltration operations can be eliminated when the extraction is carried out with an appropriate alcohol. A preferred extraction is carried out with the appropriate alcohol in the presence of an ion-pairing reagent. The process described herein allows a direct crystallization of carbapenem compounds after this type of extraction. The process eliminates the column purification step and replaces the nanofiltration step for concentration of the carbapenem compound in preparation for the crystallization step.

The invention is described herein in detail using the terms defined below unless otherwise specified.

The term "alkyl" refers to a monovalent alkane (hydrocarbon) derived radical containing from 1 to 15. carbon atoms unless otherwise defined. It may be straight or branched, and when of sufficient size, e.g., C₃₋₁₅ may be cyclic. Preferred straight or branched alkyl groups include methyl, ethyl, propyl, isopropyl, butyl and t-butyl. Preferred cycloalkyl groups include cyclopropyl, cyclopentyl and cyclohexyl.

Alkyl also includes an alkyl group substituted with a cycloalkyl group, such as cyclopropylmethyl.

Alkyl also includes a straight or branched alkyl group which contains or is interrupted by a cycloalkylene portion. Examples include the following: 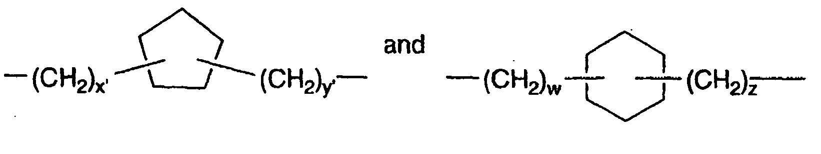 wherein: x' and y' = from 0-10; and w and z = from 0-9.

The alkylene and monovalent alkyl portion(s) of the alkyl group can be attached at any available point of attachment to the cycloalkylene portion.

When substituted alkyl is present, this refers to a straight, branched or cyclic alkyl group as defined above, substituted with 1-3 groups as defined with respect to each variable.

Aryl refers to aromatic rings e.g., phenyl, substituted phenyl and like groups as well as rings which are fused, e.g., naphthyl and the like. Aryl thus contains at least one ring having at least 6 atoms, with up to two such rings being present, containing up to 10 atoms therein, with alternating (resonating) double bonds between adjacent carbon atoms. The preferred aryl groups are phenyl and naphthyl. Aryl groups may likewise be substituted as defined below. Preferred substituted aryls include phenyl and naphthyl substituted with one to three groups, such as selected from halo, alkyl and trifluoromethyl.

The term "heteroaryl" refers to a monocyclic aromatic hydrocarbon group having 5 or 6 ring atoms, or a bicyclic aromatic group having 8 to 10 atoms, containing at least one heteroatom, O, S or N, in which a carbon or nitrogen atom is the point of attachment, and in which one additional carbon atom is optionally replaced by a heteroatom selected from O or S, and in which from 1 to 3 additional carbon atoms are optionally replaced by nitrogen heteroatoms. The heteroaryl group is optionally substituted with up to three groups.

Heteroaryl includes aromatic and partially aromatic groups which contain one or more heteroatoms. Examples of this type are thiophene, purine, imidazopyridine, pyridine, oxazole, thiazole, oxazine, pyrazole, tetrazole, imidazole, pyridine, pyrimidine, pyrazine and triazine. Examples of partially aromatic groups are tetrahydroimidazo[4,5-c]pyridine, phthalidyl and saccharinyl, as defined below.

Substituted alkyl, aryl and heteroaryl are substituted with from 1-3 groups selected from the group consisting of: halo, hydroxy, cyano, acyl, acylamino, aralkoxy, alkylsulfonyl, arylsulfonyl, alkylsulfonylamino, arylsulfonylamino, alkylaminocarbonyl, alkyl, alkoxy, aryl, aryloxy, amino, alkylamino, dialkylamino, carboxy and sulfonylamino.

Acyl as used herein refers to -C(O)C₁₋₆ alkyl and -C(O)-aryl.

Acylamino refers to the group -NHC(O)C₁₋₆ alkyl and -NHC(O)aryl.

Aralkoxy refers to the group -OC₁₋₆ alkylaryl.

Alkaryl refers to C₁₋₆ alkyl-aryl.

Alkylsulfonyl refers to the group -SO₂C₁₋₆ alkyl.

Alkylsulfonylamino refers to the group -NHSO₂C₁₋₆alkyl.

Axylsulfonylamino refers to the group -NHSO₂aryl.

Alkylaminocarbonyl refers to the group -C(O)NHC₁₋₆ alkyl.

Aryloxy refers to the group -O-aryl.

Sulfonylamino refers to the group -NHSO₃H.

Halo means Cl, F, Br and I selected on an independent basis.

Carbapenem I and II can be obtained as shown below in Flow Sheets A-1 and A-2

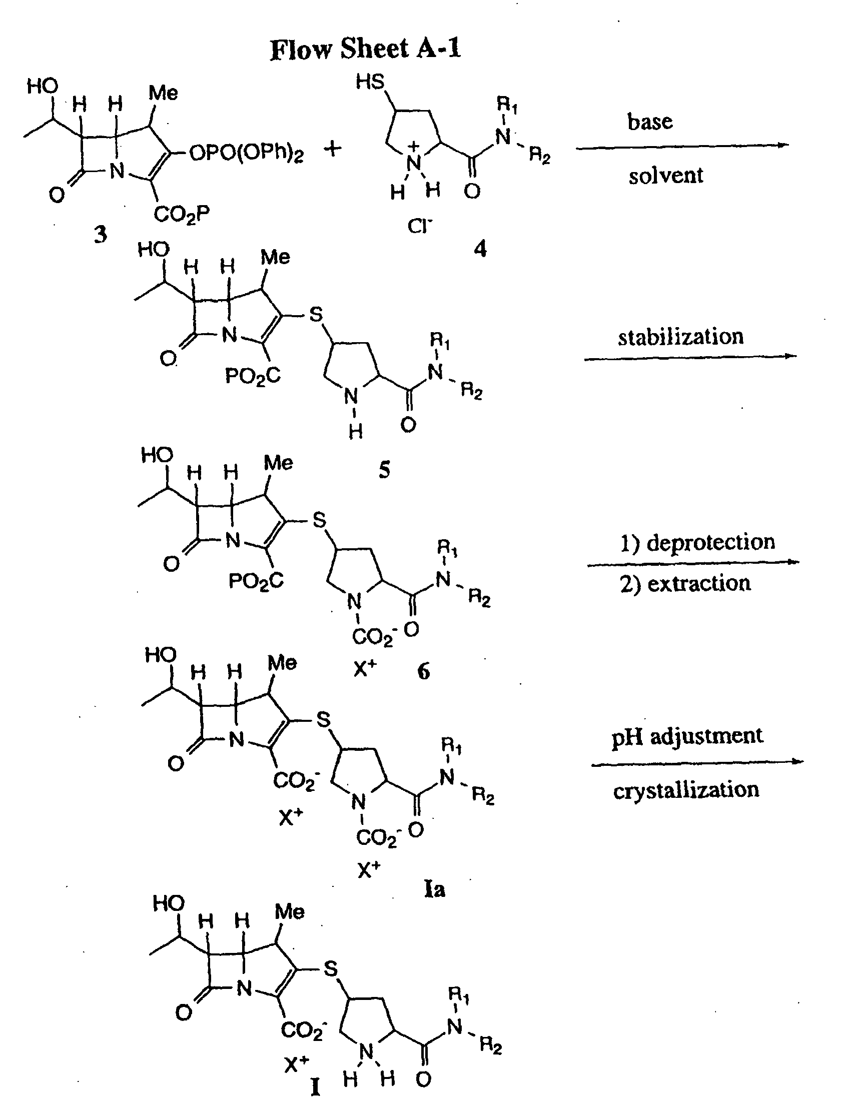

Flow sheet A-2 below provides a preferred process as it relates to 1β-methyl carbapenems.

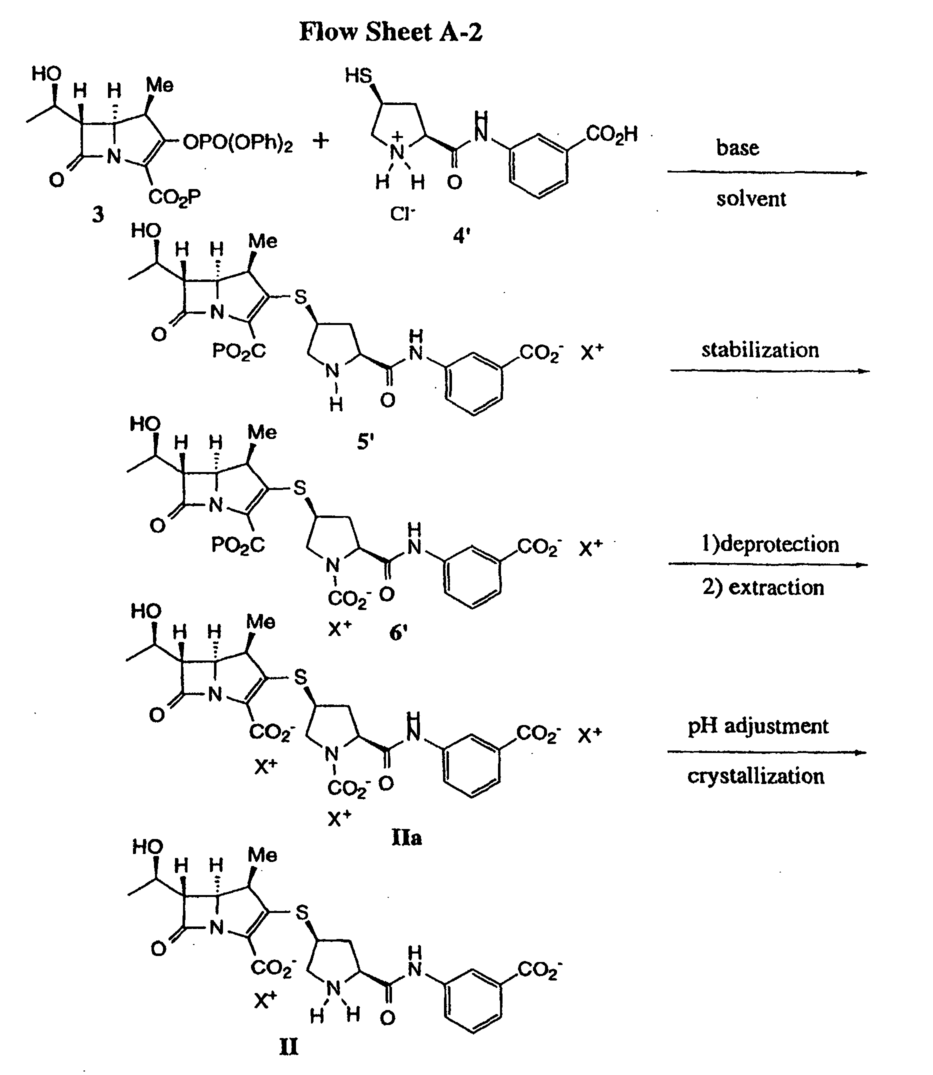

Compounds 3, 4 and 4' can be obtained in accordance with techniques such as those disclosed in U.S. Patent Nos. 5,034,384, granted on July 23, 1991; 4,994,568 granted on February 19, 1991; 4,269,772 granted on May 26, 1981; 4,350,631 granted on September 21, 1982; 4,383,946 granted on May 17, 1983; 4,414,155 granted on November 8, 1983; USSN 60/052032 (our case 19995PV), filed July 9, 1997; Tet. Let. **21,** 2783 (1980); J. Am. Chem. Soc. **108,** 6161 (1980); J. Am. Chem. Soc. **108,** 4675 (1986) and 5,478,820 granted on December 26, 1995. The teachings of these references are incorporated herein by reference. Compounds of formula I and Ia and derivatives thereof and processes thereof are disclosed in USSN 08/887849 (our case 19735), filed July 3, 1997 and USSN 60/049640 (our case 19760PV), filed June 16, 1997.

The compounds of formula 5 or 5' or salts thereof are produced by reacting the enol phosphate 3 and side chain precursor 4 or 4' in the presence of a base. This reaction is typically conducted at reduced temperature, e.g., about -30°C to about - 70°C, preferably about -40°C to about -60°C. Bases which are suitable for the above reaction include organic as well as inorganic bases. Preferred bases for use herein are secondary and tertiary amines as diisopropylamine (DIPA), dicyclohexylamine (DCHA), 2,2,6,6-tetramethylpiperidine (TMP), guanidines such as 1,1,3,3-tetramethylguanidine (TMG), N,N,N',N'N"-tetraethylcyclohexylguanidine (TECHG), N,N,N',N'-dicyclohexyldiethylguanidine (DCDEG) and amidines such as 1,8-diazabicyclo[4.3.0]undec-7-ene (DBU) and 1,5-diazabicyclo [4.3.0]non-5-ene (DBN). Most preferable bases are the guanidine bases and even more preferred is TMG.

The reaction can be conducted in an polar organic solvent, e.g., N-ethyl pyrrolidinone, N-methyl pyrrolidinone, N,N-dimethylformamide and the like. The preferred solvent is N-ethyl pyrrolidinone.

After coupling, the carbapenem is stabilized by combining the carbapenem with a carbon dioxide source. This provides a transient structure of formula 6 or 6' where X⁺ represents a charge balancing counterion and P a protecting group. Examples of carbon dioxide sources include carbon dioxide gas, bicarbonates, such as sodium and potassium bicarbonate, and carbonates such as sodium and potassium carbonate. Stabilization can be conducted according to the teachings in USSN 60/049640 (our case 19760PV), filed June 16, 1997, incorporated by reference herein.

Stabilization can be conducted under substantially neutral to slightly basic conditions, e.g., about pH 7.0 to about 8.5.

After stabilization, the carbapenem is subject to deprotection, thus removing the 3-carboxyl protecting group. The pyrrolidine nitrogen is maintained in the carbamate form during deprotection yielding Ia or IIa.

A preferred deprotection reaction is hydrogenolysis, which can be conducted using hydrogen gas or a compound which forms hydrogen.

Hydrogenolysis effectively removes the protecting group from the 3-carboxylate without substantially disrupting the β-lactam ring or the stabilized carbamate form of the pyrrolidine amine.

Hydrogenolysis is typically conducted in the presence of a metal catalyst. The preferred reaction involves H₂ gas with a palladium (Pd/C) catalyst. If necessary, base can be added. A preferred base is sodium hydroxide or sodium bicarbonate.

The stability of the pyrrolidine N-carbamate is pH dependent. The carbamate is readily converted to the unsubstituted pyrrolidine amine or ammonium salt under neutral to mildly acidic conditions. The extractions are preferably carried out under neutral or weakly basic conditions in order to maintain the stabilizing presence of the carbamate according to the teachings of WO 9745430. After the extractions are completed the pH is adjusted to produce I or II which are directly crystallized.

Carbon dioxide sources, as used herein, refer to carbon dioxide gas as well as compounds which produce carbon dioxide upon dissolution. Representative examples include carbonates and bicarbonates, such as sodium carbonate, sodium bicarbonate, potassium carbonate and potassium bicarbonate. Preferably the carbonates and bicarbonates are used. Most preferably, the carbon dioxide source is sodium bicarbonate.

The carbon dioxide source can alternatively be included in the reaction medium prior to or during the deprotection reaction.

Other protecting groups which are readily removable, i.e., they can be removed, if desired, by procedures which will not cause cleavage or other disruption of the remaining portions of the molecule, can be used. Such procedures include chemical and enzymatic hydrolysis, treatment with chemical reducing or oxidizing agents under mild conditions, treatment with fluoride ion, treatment with a transition metal catalyst and a nucleophile, and catalytic hydrogenolysis.

Examples of suitable 3-carboxyl protecting groups are: benzhydryl, o-nitrobenzyl, p-nitrobenzyl, 2-naphthylmethyl, allyl, 2-chloroallyl, benzyl, 2,2,2-trichloroethyl, trimethylsilyl, t-butyldimethylsilyl, t-butyldiphenylsilyl, 2-(trimethylsilyl)ethyl, phenacyl, p-methoxybenzyl, acetonyl, p-methoxyphenyl, 4-pyridylmethyl and t-butyl. A preferred carboxyl protecting group is p-nitrobenzyl.

Many other suitable protecting groups are known in the art. See, e.g., T.W. Greene, Protective Groups in Organic Synthesis, John Wiley & Sons, Inc., 1981 (Chapters 2 and 5).

Numerous salt-forming ions are recited in Berge, S. M., et al. **J. Pharm. Sci.** 66(1): 1-16 (1977), the teachings of which are incorporated herein by reference. The charge balancing group X, maintains overall charge neutrality. Preferably X represents a pharmaceutically acceptable salt forming cation.

Preferred salt-forming cations are selected from the group consisting of: sodium, potassium, calcium and magnesium.

More preferably the salt forming cation is a member selected from the group consisting of: Na⁺, Ca⁺² and K⁺.

The salt forming cations mentioned above provide electronic balance and overall charge neutrality. From zero to three positively charged counterions may be present, depending upon the number of charged moieties on the carbapenem. This is largely a function of pH, since at low pH, protonation of the negatively charged moieties may occur. Different counterions may also be included in the overall composition. Hence, for example, calcium and sodium could be included together in the pharmaceutical composition to provide overall charge neutrality. The counterions can thus be varied within wide limits. Generally the counterion or counterions are pharmaceutically acceptable cationic species.

The compounds formed in the present invention have asymmetric centers and occur as racemates, racemic mixtures, and as individual diastereomers. The processes of synthesizing all such isomers, including optical isomers, are included in the present invention.

In a preferred aspect of the invention a process for synthesizing a compound of the formula II 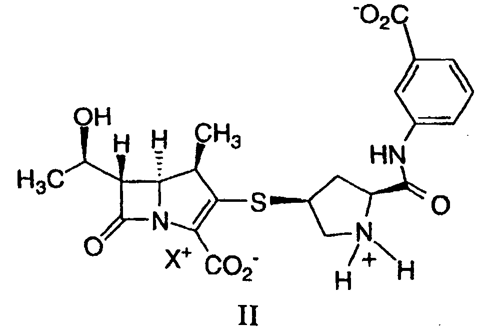 or a pharmaceutically acceptable salt or ester thereof, is disclosed,
wherein each X⁺ represents a charge balancing group,
comprising
extracting a solution containing a crude compound of formula II or IIa 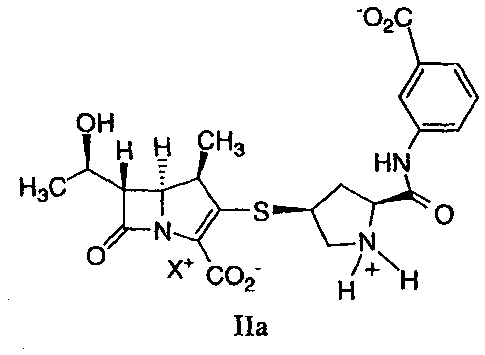 or a pharmaceutically acceptable salt thereof, wherein each X⁺ is a charge balancing group, with an alcohol, collecting and crystallizing the resultant aqueous phase to produce a crystalline compound of formula II. It is preferable that the extraction is conducted in the presence of an ion-pairing reagent and that pH of the aqueous phase is maintained between neutral and mildly basic pH. It is also preferable that the extraction is performed while II is stabilized in the form of IIa.

The alcohol useful for the present invention includes, but is not limited to, iso-amyl alcohol, tert-amyl alcohol, 1-butanol, 2-butanol, 1-octanol, 1-hexanol, 1-heptanol, cyclohexanol, 1-pentanol, cyclopentanol, 2-pentanol, 2-methyl-1-pentanol, 2-ethyl-1-butanol, 4-methyl-2-pentanol, 2,6-dimethyl-4-heptanol, 2-methylcyclohexanol, preferably 1-butanol or iso-amyl alcohol.

Preferred ion-pairing reagents for use in the present invention are lipophilic carboxylic acids, phosphoric acids, phosphinic acids, sulfonic acids and the like and their salts. Most preferred ion-pairing reagents are the sodium salts of diphenylphosphoric acid, stearic acid or dodecylbenzenesulfonic acid.

Using the process described herein the elimination of chromatographic and nanofiltration steps results in significant productivity gains reducing the overall time cycle and minimizing losses to degradation. In addition, high capital expenditures associated with chromatographic and nanofiltration steps are averted.

The invention is illustrated in connection with the following non-limiting example.

### EXAMPLE

Enolphosphate 3 (P=4-nitrobenzyl; 170 g) and sidechain 4' as its hydrochloride 1-butanol solvate (78 wt%; 108 g) were dissolved in N-methyl pyrrolidinone (NEP) [1.5 L] and cooled to -55 °C. TMG (110 g) was added slowly such that the temperature was maintained below -50 °C. The mixture was aged at -50 °C for another hour to complete the reaction and then added to a solution of sodium bicarbonate (70 g) in water (1.8 L). The temperature and pH of the resulting solution were adjusted to 5°C and 8.0, respectively, using carbon dioxide. The solution was hydrogenated in the presence of a Pd/C catalyst over a period of 1-4 hours allowing the temperature to rise from +5 °C to 20 °C. Upon completion of the reaction the catalyst was filtered.

The filtrate (4 L at pH 7.5) was extracted at 0-5 °C with 12 L of a solution prepared by dissolving diphenyl phosphoric acid (333 g) and 50% sodium hydroxide (86 g) in a mixture of iso-amyl alcohol (19.1 L) and water (1.3 L). The aqueous layer (pH 7.0) was separated and further extracted with iso-amyl alcohol (16 L). The resulting aqueous layer (0.8-1.0 L; pH 8.0-8.5) was directly used for crystallization.

Using the above aqueous solution after the extractions, the pH was adjusted from 8.0-8.5 to 5.50 using glacial acetic acid and the product was crystallized by adding methanol and 1-propanol at -10 °C, affording 90 g of the crystalline monosodium salt after filtration.

While certain preferred embodiments have been described herein in detail, numerous alternative embodiments are contemplated as falling within the scope of the appended claims.

## Claims

1. A process for the direct crystallization of a compound of the formula I: 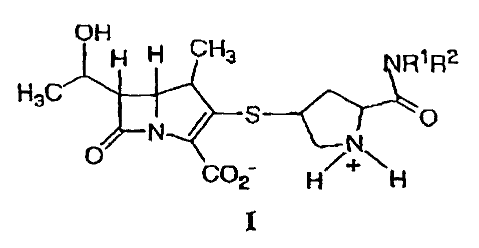 or a pharmaceutically acceptable salt thereof,
wherein R¹ and R² represent H, C₁₋₁₀ alkyl, aryl or heteroaryl, or substituted C₁₋₁₀ alkyl, aryl or heteroaryl , comprising
extracting a solution containing a crude compound of formula I or Ia: 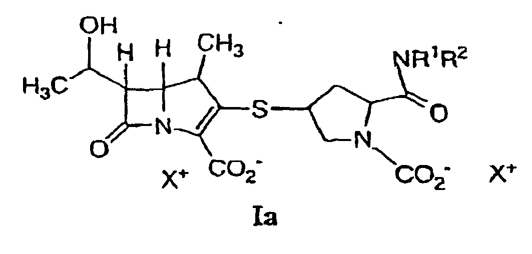 or a pharmaceutically acceptable salt thereof, wherein each X+ is a charge balancing group, and R1 and R2 are as described above with a C4-10 alcohol, collecting and crystallizing the resulting aqueous phase to produce a crystalline compound of formula I ; where "aryl" refers to phenyl or naphthyl, the term "heteroaryl" refers to a monocyclic aromatic hydrocarbon group having 5 or 6 ring atoms, or a bicyclic aromatic group having 8 to 10 atoms, containing at least one heteroatom, O, S or N, in which a carbon or nitrogen atom is the point of attachment, and in which one additional carbon atom is optionally replaced by a heteroatom selected from O or S, and in which from 1 to 3 additional carbon atoms are optionally replaced by nitrogen heteroatoms, and substituted alkyl, aryl and heteroaryl are substituted with from 1-3 groups selected from the group consisting of: halo, hydroxy, cyano, acyl, acylamino, aralkoxy, alkylsulfonyl, arylsulfonyl, alkylsulfonylamino, arylsulfonylamino, alkylaminocarbonyl, alkyl, alkoxy, aryl, aryloxy, amino, alkylamino, dialkylamino, carboxy and sulfonylamino.

2. A process according to claim 1 wherein the extraction is processed using a compound of formula Ia

3. A process for synthesizing a compound of the formula II 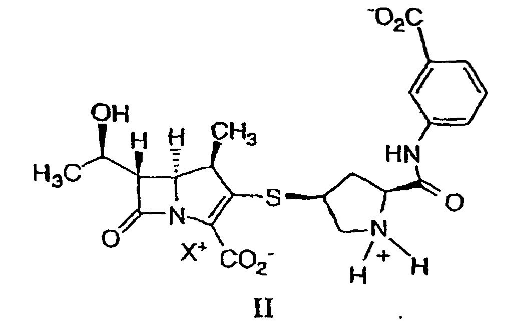 or a pharmaceutically acceptable salt thereof,
wherein X⁺ represents a charge balancing group, comprising extracting a solution containing a crude compound of formula II or IIa 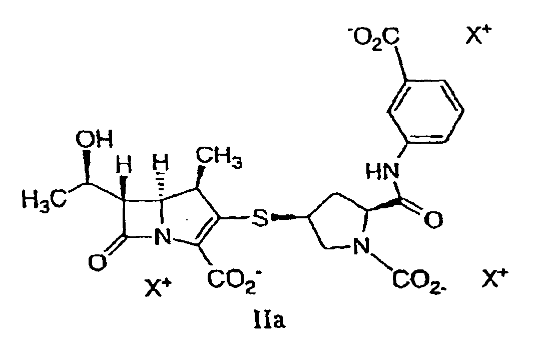 or a pharmaceutically acceptable sall thereof, wherein each X+ is a charge balancing group, with a C4-10 alcohol, collecting and crystallizing the resultant aqueous phase to produce a crystalline compound of formula II.

4. A process according to claim 1, 2 or 3 wherein the extraction is conducted with said alcohol in the presence of an ion-pairing reagent.

5. A process according to claim 3 wherein the extraction is processed using a compound of formula IIa.

6. A process for synthesizing a compound of the formula II 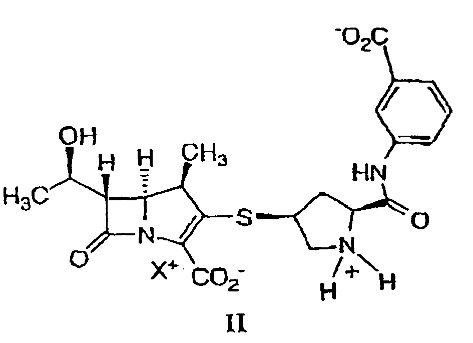 or a pharmaceutically acceptable salt thereof,
wherein X⁺ represents a charge balancing group comprising extracting a solution containing a crude compound of formula IIa 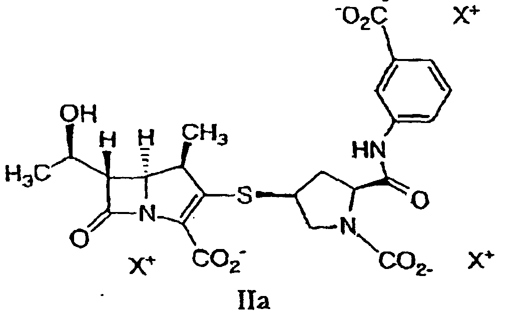 or a pharmaceutically acceptable salt thereof, wherein each X+ is a charge balancing group, with a C4-10 alcohol in the presence of an ion-pairing reagent, collecting and crystallizing the resultant aqueous phase to produce a crystalline compound of formula II.

7. A process in accordance with any preceding claim wherein the alcohol comprises iso-amyl alcohol, tert-arnyl alcohol, 1-butanol, 2-butanol, 1-octanol, 1-hexanol, 1-heptanol, cyclohexanol, 1-pentanol, cyclopentanol, 2-pentanol, 2-methyl-1-pentanal, 2-ethyl-1-butanol, 4-methyl-2-pentanol, 2,6-dimethyl-4-heptanol, or 2-methylcyclohexanol

8. A process in accordance with claim 7 wherein the alcohol is iso-amyl alcohol or 1-butanol.

9. A process in accordance with claim 4 or claim 6 wherein the ion-pairing reagent is a sodium salt of diphenylphosphoric acid, stearic acid or dodecylbenzenesulfonic acid.

## Patentansprüche

1. Ein Verfahren zur direkten Kristallisation einer Verbindung der Formel 1: 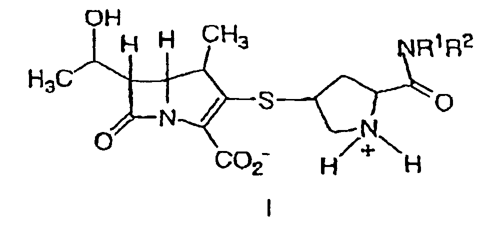 oder eines pharmazeutisch annehmbaren Salzes davon,
wobei R¹ und R² H, C₁₋₁₀-Alkyl, Aryl oder Heteroaryl oder substituiertes C₁₋₁₀-Alkyl, Aryl oder Heteroaryl bedeuten, umfassend
die Extraktion einer Lösung, die eine rohe Verbindung der Formel I oder la: 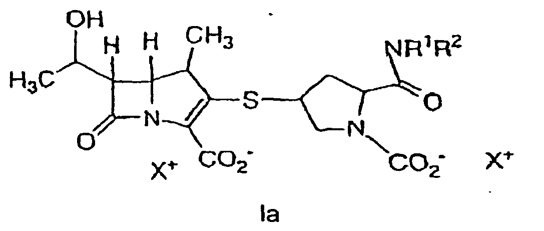 oder ein pharmazeutisch annehmbares Salz davon enthält, wobei jedes X⁺ eine ladungsausgleichende Gruppe ist und R¹ und R² wie oben beschrieben sind, mit einem C₄₋₁₀-Alkohol, das Sammeln und Kristallisieren der resultierenden wäßrigen Phase, um eine kristalline Verbindung der Formel I zu ergeben,
wobei "Aryl" Phenyl oder Naphthyl bedeutet, die Bezeichnung "Heteroaryl" eine monocyclische aromatische Kohlenwasserstoffgruppe mit 5 oder 6 Ringatomen oder eine bicyclische aromatische Gruppe mit 8 bis 10 Atomen, die wenigstens ein Heteroatom, O, S oder N, enthält, wobei ein Kohlenstoff- oder Stickstoffatom der Verknüpfungspunkt ist und wobei 1 weiteres Kohlenstoffatom gegebenenfalls durch ein Heteroatom, ausgewählt aus O oder S, ersetzt ist und wobei 1 bis 3 weitere Kohlenstoffatome gegebenenfalls durch Stickstoff-Heteroatome ersetzt sind, bedeutet,
und wobei substituiertes Alkyl, Aryl und Heteroaryl substituiert sind mit 1-3 Gruppen, ausgewählt aus der Gruppe, bestehend aus: Halogen, Hydroxy, Cyano, Acyl, Acylamino, Aralkoxy, Alkylsulfonyl, Arylsulfonyl, Alkylsulfonylamino, Arylsulfonylamino, Alkylaminocarbonyl, Alkyl, Alkoxy, Aryl, Aryloxy, Amino, Alkylamino, Dialkylamino, Carboxy und Sulfonylamino.

2. Eine Verfahren gemäß Anspruch 1, bei dem die Extraktion unter Verwendung einer Verbindung der Formel la erfolgt.

3. Ein Verfahren zur Synthese einer Verbindung der Formel II 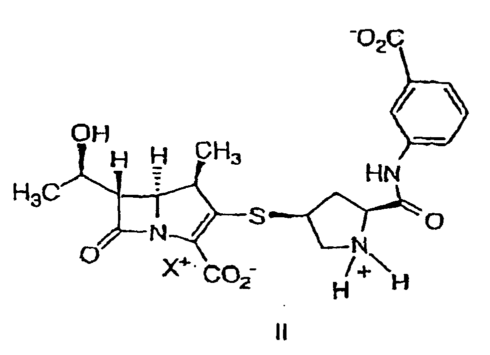 oder eines pharmazeutisch annehmbaren Salzes davon,
wobei X⁺ eine ladungsausgleichende Gruppe bedeutet,
umfassend die Extraktion einer Lösung, die eine rohe Verbindung der Formel II oder IIa 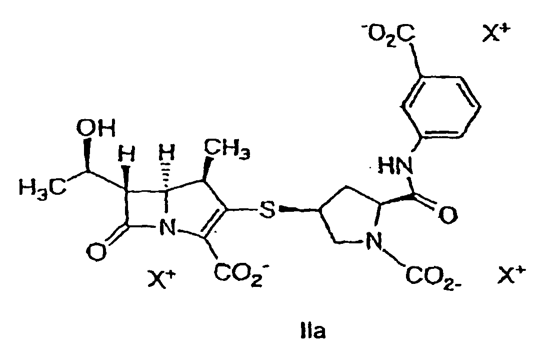 oder ein pharmazeutisch annehmbares Salz davon enthält, wobei jedes X⁺ eine ladungsausgleichende Gruppe ist, mit einem C₄₋₁₀-Alkohol, das Sammeln und Kristallisieren der resultierenden wäßrigen Phase, um eine kristalline Verbindung der Formel II zu erzeugen.

4. Ein Verfahren gemäß Anspruch 1, 2 oder 3, bei dem die Extraktion mit dem Alkohol in Gegenwart eines lonenpaar-Reagenzes durchgeführt wird.

5. Ein Verfahren gemäß Anspruch 3, bei dem die Extraktion unter Verwendung einer Verbindung der Formel IIa erfolgt.

6. Ein Verfahren zur Synthese einer Verbindung der Formel 11 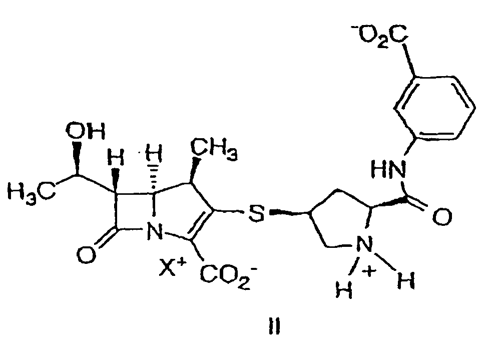 oder eines pharmazeutisch annehmbaren Salzes davon,
wobei X⁺ eine ladungsausgleichende Gruppe bedeutet,
umfassend die Extraktion einer Lösung, die eine rohe Verbindung der Formel IIa 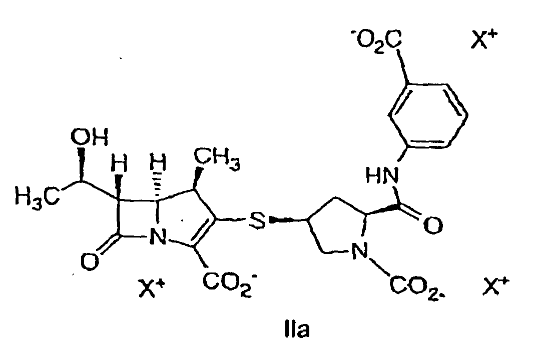 oder ein pharmazeutisch annehmbares Salz davon enthält, wobei jedes X⁺ eine ladungsausgleichende Gruppe ist, mit einem C₄₋₁₀-Alkohol in Gegenwart eines lonenpaar-Reagenzes, das Sammeln und Kristallisieren der resultierenden wäßrigen Phase, um eine kristalline Verbindung der Formel II zu erzeugen.

7. Ein Verfahren gemäß irgendeinem vorhergehenden Anspruch, bei dem der Alkohol Isoamylalkohol, tert.-Amylalkohol, 1-Butanol, 2-Butanol, 1-Octanol, 1-Hexanol, 1-Heptanol, Cyclohexanol, 1-Pentanol, Cyclopentanol, 2-Pentanol, 2-Methyl-1-pentanol, 2-Ethyl-1-butanol, 4-Methyl-2-pentanol, 2,6-Dimethyl-4-heptanol oder 2-Methylcyclohexanol umfaßt.

8. Ein Verfahren gemäß Anspruch 7, bei dem der Alkohol Isoamylalkohol oder 1-Butanol ist.

9. Ein Verfahren gemäß Anspruch 4 oder Anspruch 6, bei dem das lonenpaar-Reagenz ein Natriumsalz von Diphenylphosphorsäure, Stearinsäure oder Dodecylbenzolsulfonsäure ist.

## Revendications

1. Procédé pour la cristallisation directe d'un composé de formule I : 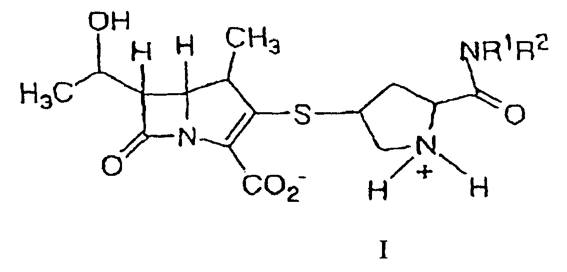 ou d'un sel pharmaceutiquement acceptable de celui-ci,
dans laquelle R¹ et R² représentent H, un alkyle en C₁ à C₁₀, un aryle ou un hétéroaryle, ou un alkyle en C₁ à C₁₀ substitué, un aryle substitué ou un hétéroaryle substitué, comprenant
l'extraction d'une solution contenant un composé brut de formule I ou Ia : 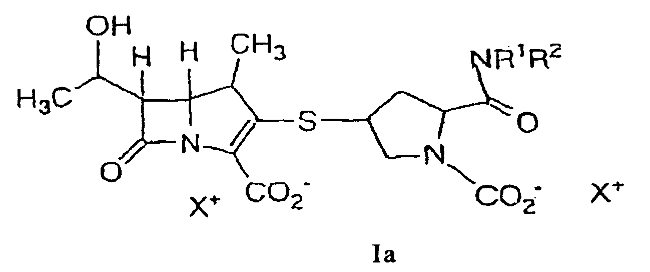 ou un sel pharmaceutiquement acceptable de celui-ci, dans laquelle chaque X+ est un groupe d'équilibrage de charge, et R1 et R2 sont tels que décrits ci-dessus avec un alcool en C4 à C10, la collecte et la cristallisation de la phase aqueuse obtenue pour produire un composé cristallin de formule I ;
où "aryle" se rapporte à un phényle ou un naphtyle, le terme "hétéroaryle" se rapporte à un groupe hydrocarbure aromatique monocyclique ayant 5 ou 6 atomes de cycle, ou un groupe aromatique bicyclique ayant 8 à 10 atomes, contenant au moins un hétéroatome O, S ou N, dans lequel un atome de carbone ou d'azote est le point d'attachement, et dans lequel un atome de carbone supplémentaire est remplacé éventuellement par un hétéroatome choisi parmi O ou S, et dans lequel de 1 à 3 atomes de carbone supplémentaires sont remplacés éventuellement par des hétéroatomes d'azote,
et un alkyle, un aryle et un hétéroaryle substitués sont substitués avec de 1 à 3 groupes choisis dans le groupe constitué par : un halogéno, un hydroxy, un cyano, un acyle, un acylamino, un aralcoxy, un alkylsulfonyle, un arylsulfonyle, un alkylsulfonylamino, un arylsulfonylamino, un alkylaminocarbonyle, un alkyle, un alcoxy, un aryle, un aryloxy, un amino, un alkylamino, un dialkylamino, un carboxy et un sulfonylamino.

2. Procédé selon la revendication 1, dans lequel l'extraction est réalisée en utilisant un composé de formule Ia.

3. Procédé pour synthétiser un composé de formule II 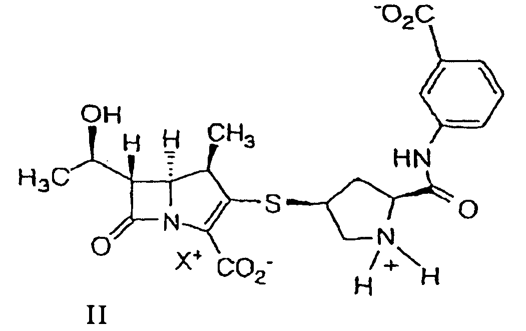 ou un sel pharmaceutiquement acceptable de celui-ci,
dans laquelle X⁺ représente un groupe d'équilibrage de charge, comprenant l'extraction d'une solution contenant un composé brut de formule II ou Iia 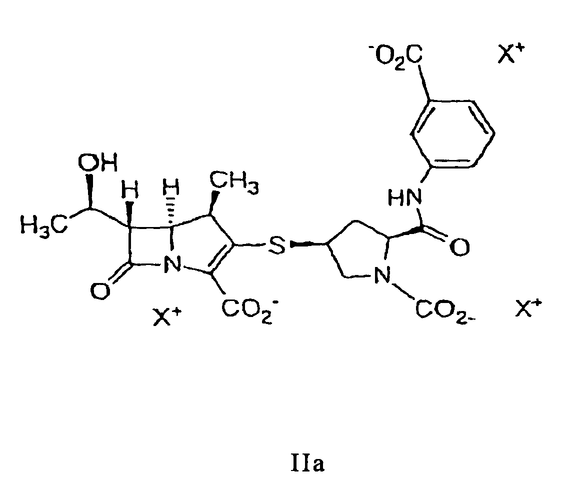 ou un sel pharmaceutiquement acceptable de celui-ci, dans laquelle chaque X+ est un groupe d'équilibrage de charge, avec un alcool en C4 à C10, la collecte et la cristallisation de la phase aqueuse résultante pour produire un composé cristallin de formule II.

4. Procédé selon la revendication 1, 2 ou 3, dans lequel l'extraction est réalisée avec ledit alcool en présence d'un réactif d'appariement d'ions.

5. Procédé selon la revendication 3, dans lequel l'extraction est réalisée en utilisant le composé de formule IIa.

6. Procédé pour synthétiser un composé de formule II 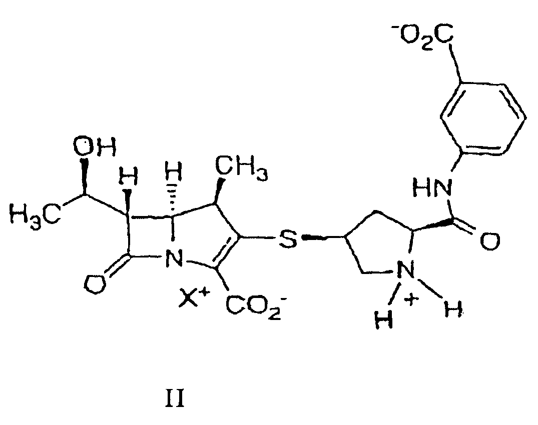 ou un sel pharmaceutiquement acceptable de celui-ci,
dans lequel X⁺ représente un groupe d'équilibrage de charge comprenant l'extraction d'une solution contenant un composé brut de formule IIa 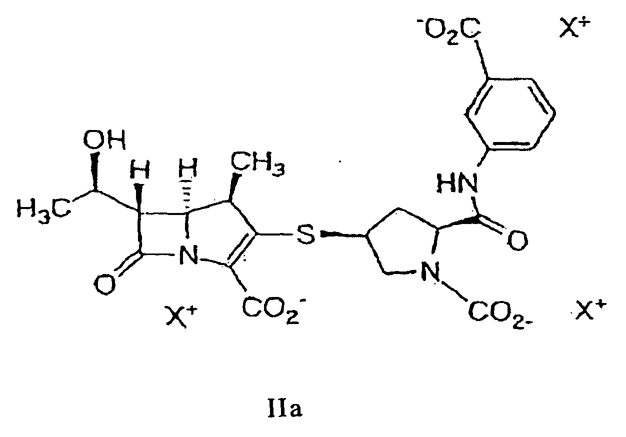 ou un sel pharmaceutiquement acceptable de celui-ci, dans laquelle chaque X⁺ est un groupe d'équilibrage de charge, avec un alcool en C4 à C10 en présence d'un réactif d'appariement d'ions, la collecte et la cristallisation de la phase aqueuse résultante pour produire un composé cristallin de formule II.

7. Procédé selon l'une quelconque des revendications précédentes, dans lequel l'alcool comprend l'alcool iso-amylique, l'alcool tert-amylique, le 1-butanol, le 2-butanol, le 1-octanol, le 1-hexanol, le 1-heptanol, le cyclohexanol, le 1-pentanol, le cyclopentanol, le 2-pentanol, le 2-méthyl-1-pentanol, le 2-éthyl-1-butanol, le 4-méthyl-2-pentanol, le 2,6-diméthyl-4-heptanol ou le 2-méthylcyclohexanol.

8. Procédé selon la revendication 7, dans lequel l'alcool est l'alcool iso-amylique ou le 1-butanol.

9. Procédé selon la revendication 4 ou la revendication 6, dans lequel le réactif d'appariement d'ions est un sel de sodium d'acide diphénylphosphorique, d'acide stéarique ou d'acide dodécylbenzènesulfonique.
